# EUROPEAN PATENT APPLICATION

(11) **EP 4 821 940 A1**
(43) Date of publication of application: **14.10.2026**
(21) Application number: 24900031.6
(22) Date of filing: 09.12.2024
(51) Int. Cl.: C07J 71/00, A61K 31/58, A61P 37/06

(54) **HIGH-EFFICACY GLUCOCORTICOID POLYMORPH, PREPARATION METHOD THEREFOR, AND USE THEREOF**

(30) Priority: 08.12.2023 CN 202311688572
(71) Applicant: Zhejiang Palo Alto Pharmaceuticals, Inc., Quzhou, Zhejiang 324099 (CN)
(72) Inventor: XI, Zhijian, Quzhou, Zhejiang 324099 (CN); LU, Chunping, Quzhou, Zhejiang 324099 (CN); CAO, Jianxin, Quzhou, Zhejiang 324099 (CN); YU, Hua, Quzhou, Zhejiang 324099 (CN)
(74) Representative: JD&P Patent Attorneys Joanna Dargiewicz & Partners
(86) International application number: PCT/CN2024/137914
(87) International publication number: WO 2025/119398

(57) **Abstract**

The present disclosure relates to novel polymorphs of a compound of formula (I), (1R,2R,3aS,3bR,10aS,10bR,118,12aS)-5,10b-difluoro-1-(((fluoromethyl)thio)carbonyl)-11-hydro xy-2,10a,12a-trimethyl-7-phenyl-1,2,3,3a,3b,7,10,10a,10b,11,12,12a-dodecahydrocyclopenta[5,6] naphtho[1,2-f]indazol-1-yl furan-2-carboxylate, and to methods for the preparation and use thereof. The polymorphs of the present disclosure have characteristics such as significant efficacy, good stability, high yield, and high purity, which facilitate the selection and design of drug administration routes and the determination of pharmaceutical formulation process parameters, thereby improving the quality of pharmaceutical production.

## Description

### TECHNICAL FIELD

The present disclosure relates to the field of medicinal chemistry, and specifically to high potency glucocorticoid polymorphs and method of preparation and use thereof.

### BACKGROUND

Currently, inflammatory and autoimmune diseases, such as asthma, arthritis, lupus, and Crohn's disease, seriously threaten human health and reduce the quality of human life. Glucocorticoids, such as prednisone, dexamethasone (DEX), and budesonide, are potent anti-inflammatory drugs widely used to treat inflammatory and autoimmune diseases. These drugs exert their physiological effects by binding to the glucocorticoid receptor (GR), a ligand-activated transcription factor of the nuclear receptor superfamily. In the absence of glucocorticoids, GR resides in the cytoplasm bound to chaperone proteins such as hsp90 and hsp70. Hormone binding induces a conformational change in GR, leading to its translocation to the nucleus where it exerts its transcriptional control activities, namely transactivation and transrepression. In transactivation, GR dimerizes, binds directly to specific glucocorticoid response elements, and then recruits coactivators to activate transcription. In transrepression, a general model is that GR binds to other transcription factors (e.g., NF-κB, AP-1) to be indirectly tethered to its binding site through protein-protein interactions. When tethered near target promoters, GR represses downstream gene expression. It is generally believed that transrepression does not require GR dimerization.

Transrepression is the primary mechanism by which glucocorticoids act as anti-inflammatory drugs. The tethering of GR to NF-κB/AP-1 promoters leads to the transcriptional repression of key downstream pro-inflammatory factors, including pro-inflammatory cytokines (e.g., TNF-α, IL-1β, IL-6), chemokines (e.g., CCL2, CCL19), and ligands associated with inflammation (e.g., COX2, MMP13, phospholipase A2). Due to their rapid action and sustained effects, glucocorticoids remain the first choice for treating inflammatory diseases. However, long-term use of glucocorticoids, especially at high doses, has numerous adverse consequences, including diabetes/glucose intolerance, hypertension, obesity, and osteoporosis. Most of these consequences are attributed to GR transactivation. For example, glucocorticoids induce genes encoding rate-limiting enzymes in the hepatic gluconeogenesis pathway, glucose-6-phosphatase and phosphoenolpyruvate carboxykinase, thereby increasing de novo glucose synthesis and ultimately leading to weight gain or diabetes. Glucocorticoids also induce Dickkopf-1 (DKK1), a key regulator of bone development, and its upregulation leads to osteoporosis and bone loss. Many side effects of glucocorticoids are generally observed in association with high-dose use. For example, a "threshold pattern" for prednisone is observed: at 7.5 mg per day, it can cause glaucoma, depression, and hypertension. These side effects are caused by GR transactivation and non-target activation of other receptors, such as the mineralocorticoid receptor (MR), whose activation causes hypertension.

Furthermore, the development of insensitivity and resistance to glucocorticoid therapy is a major issue in treating common inflammatory diseases such as chronic obstructive pulmonary disease, rheumatoid arthritis, and inflammatory bowel disease. Glucocorticoid resistance is also an unresolved problem for leukocyte cancers, especially childhood acute leukemia. Several mechanisms of glucocorticoid resistance have been identified or proposed, including alterations in kinase pathways, changes in cofactors, and receptor deletion or mutation. A common observation is a reduced affinity of the ligand for the receptor in glucocorticoid-resistant patients. Treatment of such patients with high potency glucocorticoids has shown improvement, but with diminishing effects over time.

Therefore, there is a need in the art to develop glucocorticoids with higher potency and selectivity to reduce unwanted side effects and improve therapeutic outcomes.

### SUMMARY

The present disclosure aims to provide a glucocorticoid with higher potency and selectivity to reduce unwanted side effects and improve therapeutic efficacy, specifically relating to high potency glucocorticoid polymorphs and their preparation and use.

In a first aspect of the present disclosure, a polymorph of a compound represented by formula (I) is provided, wherein the polymorph is selected from the group consisting of Form K, Form A, Form C, Form E, Form F, Form I, or Form L, 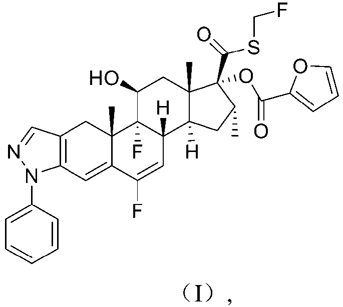 wherein the polymorph is a crystal form of (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate.

In another preferred embodiment, the XRPD pattern of the Form K comprises 3 or more (such as 4, 5, or 6) diffraction peaks having 2θ selected from the group consisting of: 7.148±0.20°, 9.336±0.20°, 9.955±0.20°, 11.239±0.20°, 12.769±0.20°, 13.826±0.20°, 14.315±0.20°, 15.581±0.20°, 15.960±0.20°, 16.748±0.20°, 17.864±0.20°, 20.050±0.20°, 21.198±0.20°, 22.587±0.20°, 22.909±0.20°, 23.725±0.20°, 24.383±0.20°, 26.611±0.20°, 27.920±0.20°.

In another preferred embodiment, the XRPD pattern of the Form K comprises 2θ diffraction peaks selected from the group consisting of: 7.148±0.20°, 9.336±0.20°, 9.955±0.20°, 11.239±0.20°, 12.769±0.20°, 13.826±0.20°, 14.315±0.20°, 15.581±0.20°, 15.960±0.20°, 16.748±0.20°, 17.864±0.20°, 20.050±0.20°, 21.198±0.20°, 22.587±0.20°, 22.909±0.20°, 23.725±0.20°, 24.383±0.20°, 26.611±0.20°, 27.920±0.20°.

In another preferred embodiment, the Form K further comprises 2θ diffraction peaks selected from the group consisting of: 7.750±0.20°, 11.639±0.20°, 25.100±0.20°, 29.369±0.20°, 30.269±0.20°, 36.306±0.20°, 36.978±0.20°.

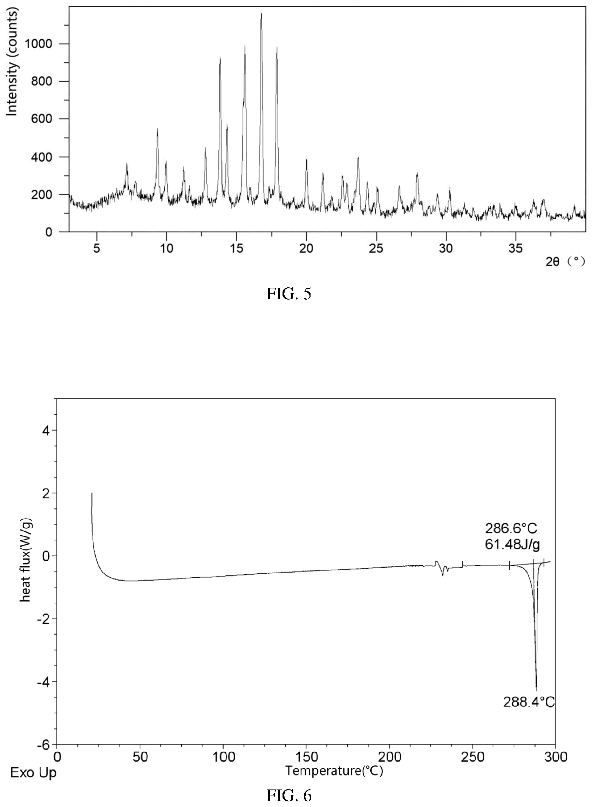

In another preferred embodiment, the Form K further comprises one or more features selected from the group consisting of:
(i) The XRPD pattern of Form K is essentially as depicted in FIG. 5;
(ii) The DSC thermogram of Form K is essentially as depicted in FIG. 6;
(iii) The DSC thermogram of Form K shows an endothermic peak in the range of 286.4°C-290.4°C;
(iv) Form K is an anhydrate.

In another preferred embodiment, the preparation method for Form K comprises: a) suspending and stirring the starting material in MeOH at room temperature; b) isolating the solid by suction filtration to obtain the target Form K.

In another preferred embodiment, the XRPD pattern of the Form A comprises 3 or more (such as 4, 5, or 6) diffraction peaks having 2θ selected from the group consisting of: 5.636±0.20°, 8.210±0.20°, 11.248±0.20°, 13.049±0.20°, 16.898±0.20°, 17.722±0.20°.

In another preferred embodiment, the XRPD pattern of the Form A comprises 2θ diffraction peaks selected from the group consisting of: 5.636±0.20°, 8.210±0.20°, 11.248±0.20°, 13.049±0.20°, 16.898±0.20°, 17.722±0.20°.

In another preferred embodiment, the XRPD pattern of the Form A comprises 2θ diffraction peaks selected from the group consisting of: 8.684±0.20°, 14.791±0.20°, 15.592±0.20°, 17.872±0.20°, 18.721±0.20°, 21.127±0.20°, 22.543±0.20°, 24.687±0.20°, 25.757±0.20°.

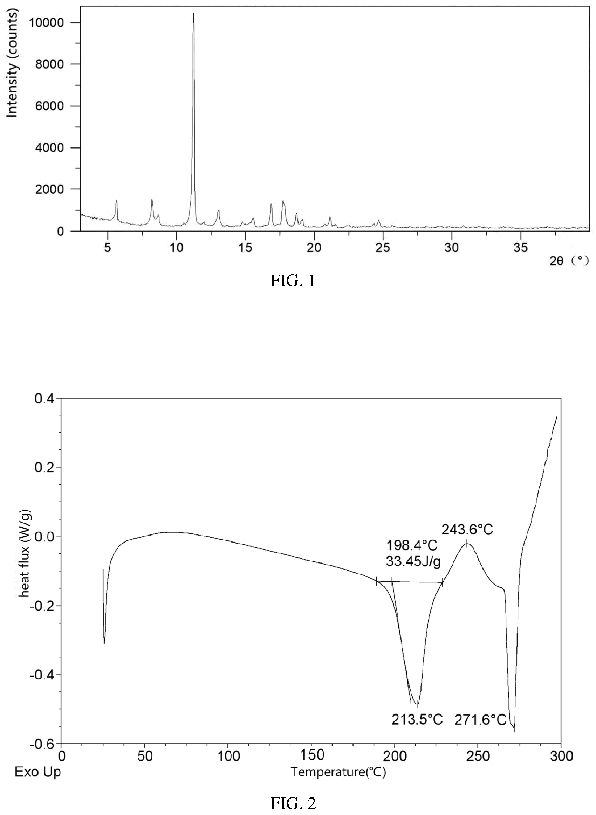

In another preferred embodiment, the Form A further comprises one or more features selected from the group consisting of:
(i) The XRPD pattern of Form A is essentially as depicted in FIG. 1;
(ii) The DSC thermogram of Form A is essentially as depicted in FIG. 2;
(iii) The DSC thermogram of Form A shows endothermic peaks in the ranges of
211.5°C-215.5°C and 269.6°C-273.6°C.

In another preferred embodiment, the preparation method for Form A comprises: a) suspending and stirring the starting material in MeOH at 5°C; b) isolating the solid by suction filtration to obtain the target Form A.

In another preferred embodiment, the XRPD pattern of the Form C comprises 3 or more (such as 4, 5, or 6) diffraction peaks having 2θ selected from the group consisting of: 6.463±0.20°, 7.402±0.20°, 8.988±0.20°, 12.000±0.20°, 12.898±0.20°, 13.088±0.20°, 13.611±0.20°, 14.328±0.20°, 16.233±0.20°, 17.205±0.20°, 19.794±0.20°, 21.032±0.20°.

In another preferred embodiment, the XRPD pattern of the Form C comprises 2θ diffraction peaks selected from the group consisting of: 6.463±0.20°, 7.402±0.20°, 8.988±0.20°, 12.000±0.20°, 12.898±0.20°, 13.088±0.20°, 13.611±0.20°, 14.328±0.20°, 16.233±0.20°, 17.205±0.20°, 19.794±0.20°, 21.032±0.20°.

In another preferred embodiment, the XRPD pattern of the Form C comprises 2θ diffraction peaks selected from the group consisting of: 10.812±0.20°, 23.376±0.20°, 24.173±0.20°, 24.815±0.20°, 26.038±0.20°, 29.921±0.20°.

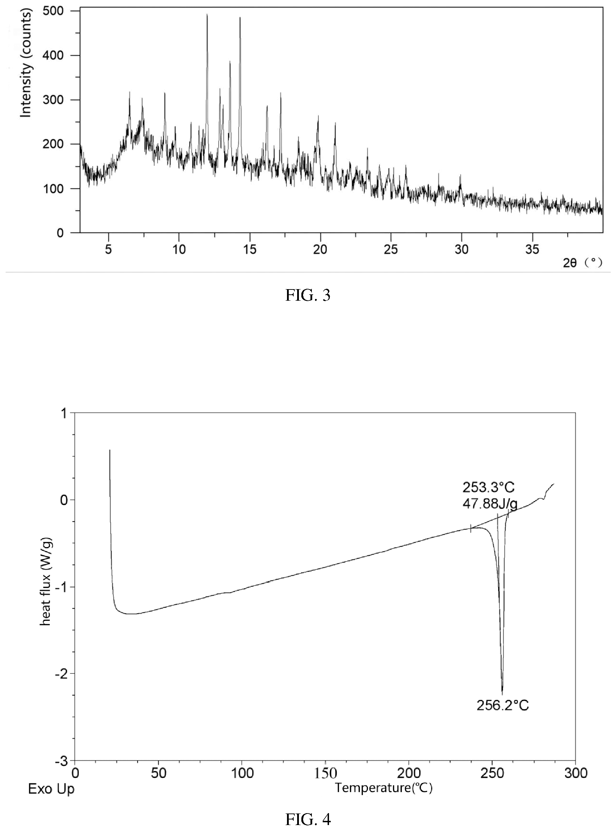

In another preferred embodiment, the Form C further comprises one or more features selected from the group consisting of:
(i) The XRPD pattern of Form C is essentially as depicted in FIG. 3;
(ii) The DSC thermogram of Form C is essentially as depicted in FIG. 4;
(iii) The DSC thermogram of Form C shows an endothermic peak in the range of 254.2°C-258.2°C.

In another preferred embodiment, the preparation method for Form C comprises: a) suspending and stirring the starting material in EtOAc/n-Heptane (1:3, v/v) at room temperature; b) isolating the solid by suction filtration to obtain the target Form C.

In another preferred embodiment, the XRPD pattern of the Form E comprises 3 or more (such as 4, 5, or 6) diffraction peaks having 2θ selected from the group consisting of: 4.773±0.20°, 7.773±0.20°, 13.086±0.20°, 21.528±0.20°.

In another preferred embodiment, the Form E comprises 2θ diffraction peaks selected from the group consisting of: 4.773±0.20°, 7.773±0.20°, 13.086±0.20°, 21.528±0.20°.

In another preferred embodiment, the Form E further comprises 2θ diffraction peaks selected from the group consisting of: 16.844±0.20°, 18.108±0.20°.

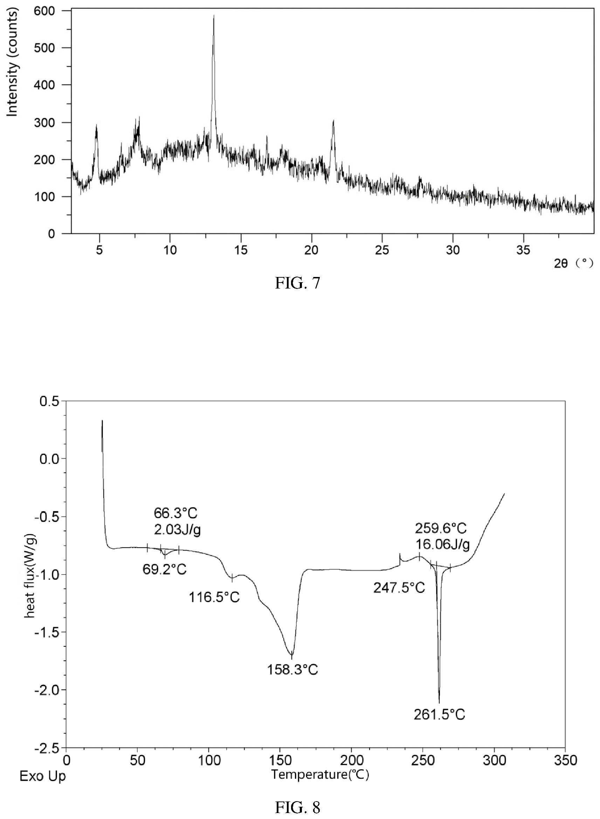

In another preferred embodiment, the Form E further comprises one or more features selected from the group consisting of:
(i) The XRPD pattern of Form E is essentially as depicted in FIG. 7;
(ii) The DSC thermogram of Form E is essentially as depicted in FIG. 8;
(iii) The DSC thermogram of Form E shows endothermic peaks in the ranges of 67.2°C-71.2°C, 114.5°C-118.5°C, 156.3°C-160.3°C, and 259.5°C-263.5°C.

In another preferred embodiment, the preparation method for Form E comprises: a) suspending and stirring the starting material in IPA at 5°C; b) isolating the solid by suction filtration to obtain the target Form E.

In another preferred embodiment, the XRPD pattern of the Form F comprises 3 or more (such as 4, 5, or 6) diffraction peaks having 2θ selected from the group consisting of: 12.132±0.20°, 12.389±0.20°, 12.809±0.20°, 15.086±0.20°, 17.853±0.20°, 18.413±0.20°, 18.627±0.20°, 22.262±0.20°, 24.325±0.20°, 25.191±0.20°.

In another preferred embodiment, the Form F comprises 2θ diffraction peaks selected from the group consisting of: 12.132±0.20°, 12.389±0.20°, 12.809±0.20°, 15.086±0.20°, 17.853±0.20°, 18.413±0.20°, 18.627±0.20°, 22.262±0.20°, 24.325±0.20°, 25.191±0.20°.

In another preferred embodiment, the XRPD pattern of the Form F further comprises 2θ diffraction peaks selected from the group consisting of: 6.310±0.20°, 13.849±0.20°, 16.717±0.20°, 17.088±0.20°, 19.179±0.20°, 19.493±0.20°, 20.259±0.20°, 23.851±0.20°, 26.158±0.20°, 28.159±0.20°, 28.556±0.20°, 34.014±0.20°.

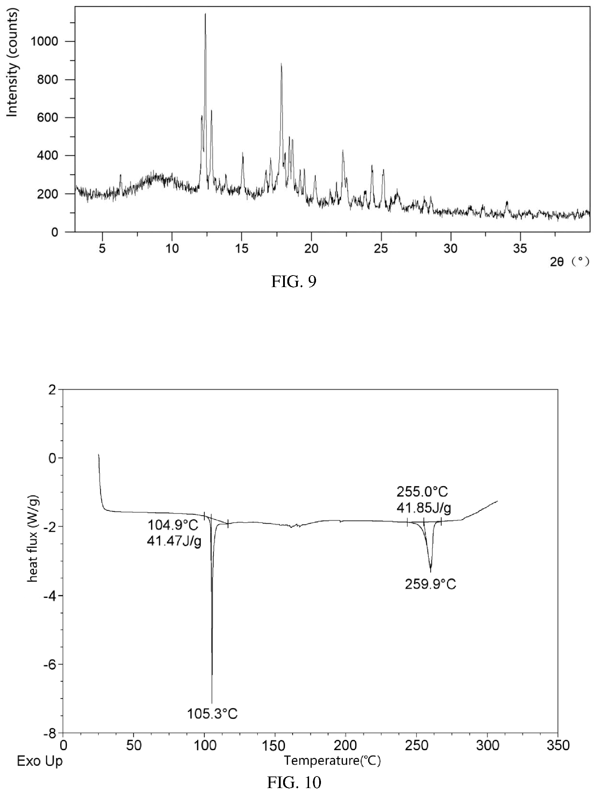

In another preferred embodiment, the Form F further comprises one or more features selected from the group consisting of:
(i) The XRPD pattern of Form F is essentially as depicted in FIG. 9;
(ii) The DSC thermogram of Form F is essentially as depicted in FIG. 10;
(iii) The DSC thermogram of Form F shows endothermic peaks in the ranges of 103.3°C-107.3°C and 257.9°C-261.9°C.

In another preferred embodiment, the preparation method for Form F comprises: a) suspending and stirring the starting material in DMF/H₂O (2:1, v/v) at room temperature; b) isolating the solid by suction filtration to obtain the target Form F.

In another preferred embodiment, the XRPD pattern of the Form I comprises 3 or more (such as 4, 5, or 6) diffraction peaks having 2θ selected from the group consisting of: 5.120±0.20°, 6.627±0.20°, 12.714±0.20°, 15.388±0.20°, 18.439±0.20°, 18.978±0.20°, 25.496±0.20°.

In another preferred embodiment, the XRPD pattern of the Form I comprises 2θ diffraction peaks selected from the group consisting of: 5.120±0.20°, 6.627±0.20°, 12.714±0.20°, 15.388±0.20°, 18.439±0.20°, 18.978±0.20°, 25.496±0.20°.

In another preferred embodiment, the polymorph is Form I, wherein the XRPD pattern of Form I comprises at least 2θ diffraction peaks selected from the group consisting of: 5.120±0.20°, 6.627±0.20°, 12.714±0.20°, 18.439±0.20°, 18.978±0.20°.

In another preferred embodiment, the XRPD pattern of the Form I further comprises 2θ diffraction peaks selected from the group consisting of: 13.197±0.20°, 14.183±0.20°, 19.535±0.20°, 22.701±0.20°, 34.482±0.20°.

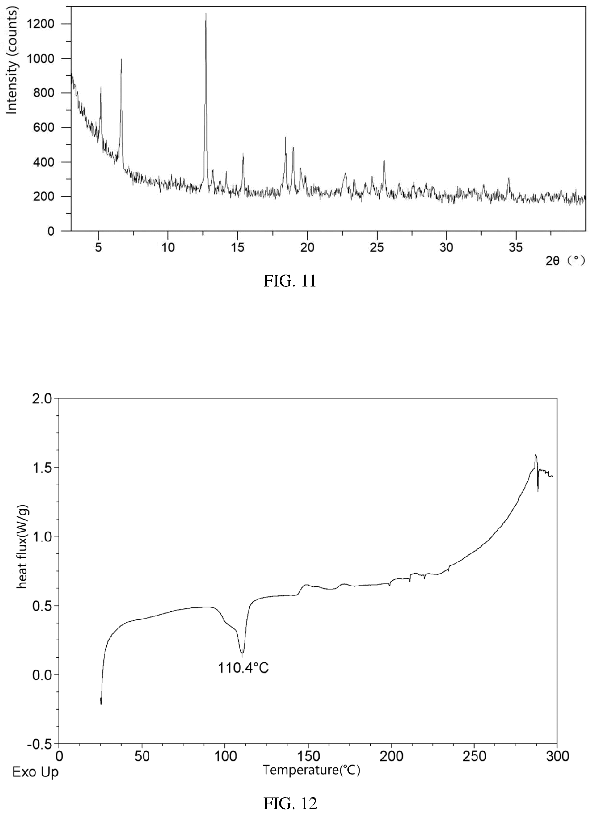

In another preferred embodiment, the Form I further comprises one or more features selected from the group consisting of:
(i) The XRPD pattern of Form I is essentially as depicted in FIG. 11;
(ii) The DSC thermogram of Form I is essentially as depicted in FIG. 12;
(iii) The DSC thermogram of Form I shows an endothermic peak in the range of 108.4°C-112.4°C.

In another preferred embodiment, the preparation method for Form I comprises: a) dissolving the starting material in DMF/1-Butanol (1:1, v/v) and slowly cooling; b) isolating the solid by suction filtration to obtain the target Form I.

In another preferred embodiment, the XRPD pattern of the Form L comprises 3 or more (such as 4, 5, or 6) diffraction peaks having 2θ selected from the group consisting of: 6.586±0.20°, 8.336±0.20°, 12.065±0.20°, 12.568±0.20°, 13.569±0.20°, 14.503±0.20°, 15.950±0.20°, 17.259±0.20°, 18.918±0.20°, 23.483±0.20°.

In another preferred embodiment, the XRPD pattern of the Form L comprises 2θ diffraction peaks selected from the group consisting of: 6.586±0.20°, 8.336±0.20°, 12.065±0.20°, 12.568±0.20°, 13.569±0.20°, 14.503±0.20°, 15.950±0.20°, 17.259±0.20°, 18.918±0.20°, 23.483±0.20°.

In another preferred embodiment, the XRPD pattern of the Form L further comprises 2θ diffraction peaks selected from the group consisting of: 5.867±0.20°, 9.324±0.20°, 10.609±0.20°, 13.115±0.20°, 17.783±0.20°, 18.545±0.20°, 19.713±0.20°, 20.940±0.20°, 21.336±0.20°, 22.348±0.20°, 28.165±0.20°, 31.875±0.20°.

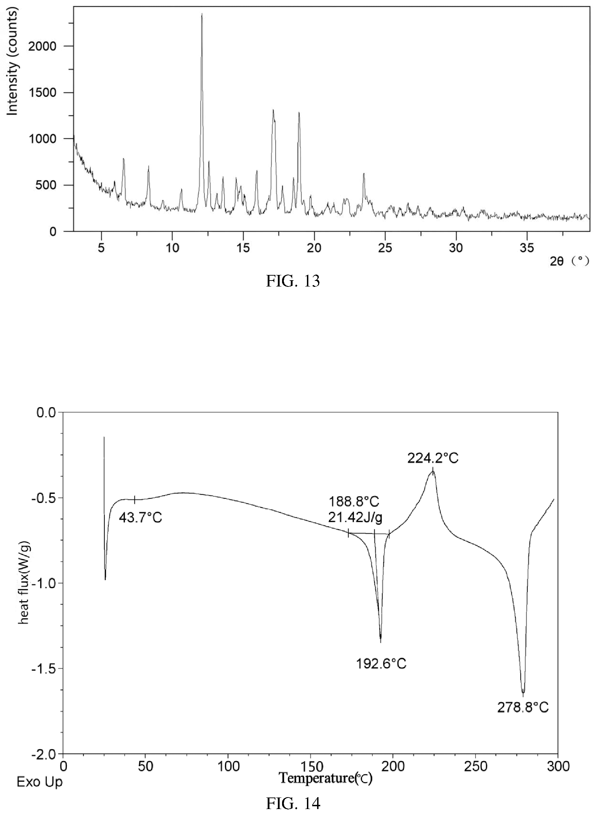

In another preferred embodiment, the Form L further comprises one or more features selected from the group consisting of:
(i) The XRPD pattern of Form L is essentially as depicted in FIG. 13;
(ii) The DSC thermogram of Form L is essentially as depicted in FIG. 14;
(iii) The DSC thermogram of Form L shows endothermic peaks in the ranges of 190.6°C-194.6°C and 276.8°C-280.8°C.

In another preferred embodiment, the preparation method for Form L comprises: a) suspending and stirring a mixed sample of Forms A/C/K in EtOAc at room temperature with rotational mixing; b) isolating the solid by suction filtration to obtain the target Form L.

In a second aspect of the present disclosure, a pharmaceutical composition is provided, comprising the polymorph of formula (I) according to the first aspect, and optionally, a pharmaceutically acceptable carrier.

In another preferred embodiment, the composition is suitable for oral, injectable, nasal spray, oral inhalation aerosol, and topical skin application routes of administration.

In another preferred embodiment, the composition is suitable for nasal spray, oral inhalation aerosol, and topical skin application routes of administration.

In another preferred embodiment, the composition can be formulated as tablets, injections, parenteral preparations, sprays, aerosols, and creams.

In another preferred embodiment, the composition can be formulated as sprays, aerosols, and creams.

In a third aspect of the present disclosure, a use of the polymorph according to the first aspect is provided, for the manufacture of a medicament for the treatment or prevention of rhinitis, asthma, or neurodermatitis.

In another preferred embodiment, it is for the manufacture of a medicament for the treatment or prevention of rhinitis, asthma, or neurodermatitis.

It should be understood that within the scope of the present disclosure, the above-mentioned technical features and the technical features specifically described below (e.g., in the Examples) can be combined with each other to form new or preferred technical solutions. Due to space limitations, they are not repeated here.

### BRIEF DESCRIPTION OF THE DRAWINGS

FIG. 1 shows the XRPD pattern of Form A.
FIG. 2 shows the DSC thermogram of Form A.
FIG. 3 shows the XRPD pattern of Form C.
FIG. 4 shows the DSC thermogram of Form C.
FIG. 5 shows the XRPD pattern of Form K.
FIG. 6 shows the DSC thermogram of Form K.
FIG. 7 shows the XRPD pattern of Form E.
FIG. 8 shows the DSC thermogram of Form E.
FIG. 9 shows the XRPD pattern of Form F.
FIG. 10 shows the DSC thermogram of Form F.
FIG. 11 shows the XRPD pattern of Form I.
FIG. 12 shows the DSC thermogram of Form I.
FIG. 13 shows the XRPD pattern of Form L.
FIG. 14 shows the DSC thermogram of Form L.
FIG. 15 shows the XRPD overlay of the suspension competition of Forms A/C/K in the MeOH system at room temperature.
FIG. 16 shows the XRPD overlay of the suspension competition of Forms A/C/K in the EtOAc system at room temperature.
FIG. 17 shows the DVS isotherm plot of Form K.
FIG. 18 shows the XRPD overlay of Form K before and after DVS testing.
FIG. 19 shows the XRPD overlay of Form K before and after storage.

### DETAILED DESCRIPTION

The present inventors, through extensive and in-depth research and extensive experimental screening, have unexpectedly developed for the first time polymorphs of the glucocorticoid drug (1R,2R,3aS,3bR,10aS,10bR,118,12a5)-5,10b-difluoro-1-(((fluoromethyl)thio)carbonyl)-11-hydro xy-2,10a,12a-trimethyl-7-phenyl-1,2,3,3a,3b,7,10,10a,10b,11,12,12a-dodecahydrocyclopenta[5,6] naphtho[1,2-f]indazol-1-yl furan-2-carboxylate, and methods for their preparation, the polymorphs being selected from the group consisting of Form A, Form C, Form K, Form E, Form F, Form I, or Form L. The glucocorticoid polymorphs of the present disclosure (especially Form K) possess higher potency and higher selectivity, capable of reducing side effects during treatment and improving therapeutic outcomes. Based on this, the present disclosure has been completed.

### TERMINOLOGY

### High Potency Glucocorticoid Polymorphs

(1R,2R,3aS,3bR,10aS,10bR,11S,12aS)-5,10b-difluoro-1-(((fluoromethyl)thio)carbonyl)-11-hydroxy-2,10a,12a-trimethyl-7-phenyl-1,2,3,3a,3b,7,10,10a,10b,11,12,12a-dodecahydrocyclopent a[5,6]naphtho[1,2-f]indazol-1-yl furan-2-carboxylate (I) has the following structure:

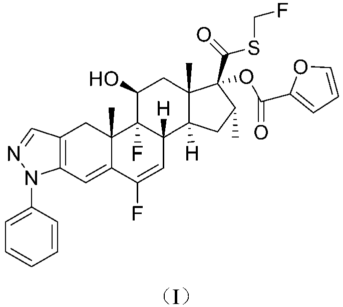

Potency and efficacy are two key pharmacokinetic parameters for glucocorticoids. While efficacy is the maximum activity achievable by a given drug, usually at maximum concentration, potency is the concentration of a given drug required to achieve half-maximal activity (EC50). For two glucocorticoids with the same efficacy, a high potency glucocorticoid will require a lower dose to achieve the same therapeutic effect. Importantly, glucocorticoids may have different potencies for transactivation and transrepression. For example, gene induction by GR via DEX requires 5- to 6-fold higher glucocorticoid concentrations than gene repression. This differential response offers an opportunity to develop a high potency glucocorticoid that can be used at low doses to achieve complete inhibition of inflammatory signals while having minimal transactivation activity and side effects.

Currently, drug polymorphism has become an indispensable and important part of the drug research process and the quality control and testing of finished pharmaceutical products. Research on drug polymorphism aids in the selection of biological activity of new drug compounds, helps improve bioavailability and clinical efficacy, facilitates the selection and design of drug administration routes, and the determination of pharmaceutical formulation process parameters, thereby improving the quality of pharmaceutical production. The bioavailability of the same drug can vary significantly between different crystal forms. For the same drug, some crystal forms may possess higher biological activity than others. Obtaining a crystal form with higher biological activity and greater suitability for pharmaceutical applications is a technical problem that the pharmaceutical field has long sought to solve.

The present disclosure provides polymorphs of (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate.

In the present disclosure, (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate is a novel high potency glucocorticoid. Based on the determined X-ray structures of the glucocorticoid receptor (GR) ligand-binding domain (LBD) bound to cortisol and mometasone furoate (MF), the inventors have obtained, through structure-based design, a novel glucocorticoid with greatly improved potency. High potency glucocorticoids allow for minimization of side effects, achieving the same therapeutic effect at lower doses.

In another preferred embodiment, the polymorph is Form A, wherein the XRPD pattern of Form A comprises at least diffraction peaks at 2θ±0.20° of: 5.636, 8.210, 11.248, 13.049, 16.898, 17.722.

Preferably, the XRPD pattern of the (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate Form A comprises at least diffraction peaks at 2θ±0.20° of: 5.636, 8.210, 8.684, 11.248, 13.049, 16.898, 17.722, 17.872, 21.127, 24.687. More preferably, it further comprises diffraction peaks at 2θ±0.20° of: 14.791, 15.592, 18.721, 22.543, 25.757.

Particularly preferably, the Form A has an XRPD pattern essentially as shown in FIG. 1.

In another preferred embodiment, the polymorph is Form A, and differential thermal analysis of Form A shows endothermic peaks at 198.4°C (onset) and 271.6°C (peak), and an exothermic peak at 243.6°C (peak).

In another preferred embodiment, the polymorph is Form A, and Form A has a DSC thermogram essentially as shown in FIG. 2.

In a specific embodiment of the present disclosure, a method for preparing Form A is also provided, comprising a) suspending and stirring the starting material in MeOH at 5°C; b) isolating the solid by suction filtration to obtain the target Form A.

In another preferred embodiment, the polymorph is Form C, wherein the XRPD pattern of Form C comprises at least diffraction peaks at 2θ±0.20° of: 8.988, 12.000, 12.898, 13.088, 13.611, 14.328, 16.233, 17.205, 19.794.

Preferably, the XRPD pattern of the (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate Form C comprises at least diffraction peaks at 2θ±0.20° of: 6.463, 7.402, 8.988, 12.000, 12.898, 13.088, 13.611, 14.328, 16.233, 17.205, 19.794, 21.032. More preferably, it further comprises diffraction peaks at 2θ±0.20° of: 10.812, 23.376, 24.173, 24.815, 26.038, 29.921.

Particularly preferably, the Form C has an XRPD pattern essentially as shown in FIG. 3.

In another preferred embodiment, the polymorph is Form C, and differential thermal analysis of Form C shows a sharp endothermic melting peak at 256.2°C (peak).

In another preferred embodiment, the polymorph is Form C, and Form C has a DSC thermogram essentially as shown in FIG. 4.

In a specific embodiment of the present disclosure, a method for preparing Form C is also provided, comprising a) suspending and stirring the starting material in EtOAc/n-Heptane (1:3, v/v) at room temperature; b) isolating the solid by suction filtration to obtain the target Form C.

In another preferred embodiment, the polymorph is Form K, wherein the XRPD pattern of Form K comprises at least diffraction peaks at 2θ±0.20° of: 9.336, 9.955, 12.769, 13.826, 14.315, 15.581, 15.960, 16.748, 17.864, 20.050, 21.198, 22.587, 22.909, 23.725, 27.920.

Preferably, the XRPD pattern of the (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate Form K comprises at least diffraction peaks at 2θ±0.20° of: 7.148, 9.336, 9.955, 11.239, 12.769, 13.826, 14.315, 15.581, 15.960, 16.748, 17.864, 20.050, 21.198, 22.587, 22.909, 23.725, 24.383, 26.611, 27.920. More preferably, it further comprises diffraction peaks at 2θ±0.20° of: 7.750, 11.639, 25.100, 29.369, 30.269, 36.306, 36.978.

Particularly preferably, the Form K has an XRPD pattern essentially as shown in FIG. 5.

In another preferred embodiment, the polymorph is Form K, and differential thermal analysis of Form K shows a sharp endothermic melting peak at 288.4°C (peak).

In another preferred embodiment, the polymorph is Form K, and Form K has a DSC thermogram essentially as shown in FIG. 6.

In a specific embodiment of the present disclosure, a method for preparing Form K is also provided, comprising a) suspending and stirring the starting material in MeOH at room temperature; b) isolating the solid by suction filtration to obtain the target Form K.

In another preferred embodiment, the polymorph is Form E, wherein the XRPD pattern of Form E comprises at least diffraction peaks at 2θ±0.20° of: 4.773, 13.086, 21.528.

Preferably, the XRPD pattern of the (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate Form E comprises at least diffraction peaks at 2θ±0.20° of: 4.773, 7.773, 13.086, 21.528. More preferably, it further comprises diffraction peaks at 2θ±0.20° of: 16.844, 18.108.

Particularly preferably, the Form E has an XRPD pattern essentially as shown in FIG. 7.

In another preferred embodiment, the polymorph is Form E, and differential thermal analysis of Form E shows endothermic peaks at 69.2°C, 116.5°C, 158.3°C, and 261.5°C (peak), and an exothermic peak at 259.6°C (onset).

In another preferred embodiment, the polymorph is Form E, and Form E has a DSC thermogram essentially as shown in FIG. 8.

In a specific embodiment of the present disclosure, a method for preparing Form E is also provided, comprising a) suspending and stirring the starting material in IPA at 5°C; b) isolating the solid by suction filtration to obtain the target Form E.

In another preferred embodiment, the polymorph is Form F, wherein the XRPD pattern of Form F comprises at least diffraction peaks at 2θ±0.20° of: 12.132, 12.389, 12.809, 18.627, 22.262, 24.325, 25.191.

Preferably, the XRPD pattern of the (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate Form F comprises at least diffraction peaks at 2θ±0.20° of: 12.132, 12.389, 12.809, 15.086, 17.853, 18.413, 18.627, 22.262, 24.325, 25.191. More preferably, it further comprises diffraction peaks at 2θ±0.20° of: 6.310, 13.849, 16.717, 17.088, 19.179, 19.493, 20.259, 23.851, 26.158, 28.159, 28.556, 34.014.

Particularly preferably, the Form F has an XRPD pattern essentially as shown in FIG. 9.

In another preferred embodiment, the polymorph is Form F, and differential thermal analysis of Form F shows endothermic peaks at 105.3°C and 259.9°C (peak).

In another preferred embodiment, the polymorph is Form F, and Form F has a DSC thermogram essentially as shown in FIG. 10.

In a specific embodiment of the present disclosure, a method for preparing Form F is also provided, comprising a) suspending and stirring the starting material in DMF/H₂O (2:1, v/v) at room temperature; b) isolating the solid by suction filtration to obtain the target Form F.

In another preferred embodiment, the polymorph is Form I, wherein the XRPD pattern of Form I comprises at least diffraction peaks at 2θ±0.20° of: 5.120, 6.627, 12.714, 18.439, 18.978.

Preferably, the XRPD pattern of the (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate Form I comprises at least diffraction peaks at 2θ±0.20° of: 5.120, 6.627, 12.714, 15.388, 18.439, 18.978, 25.496. More preferably, it further comprises diffraction peaks at 2θ±0.20° of: 13.197, 14.183, 19.535, 22.701, 34.482.

Particularly preferably, the Form I has an XRPD pattern essentially as shown in FIG. 11.

In another preferred embodiment, the polymorph is Form I, and differential thermal analysis of Form I shows an endothermic peak at 110.4°C (peak).

In another preferred embodiment, the polymorph is Form I, and Form I has a DSC thermogram essentially as shown in FIG. 12.

In a specific embodiment of the present disclosure, a method for preparing Form I is also provided, comprising a) dissolving the starting material in DMF/1-Butanol (1:1, v/v) and slowly cooling; b) isolating the solid by suction filtration to obtain the target Form I.

In another preferred embodiment, the polymorph is Form L, wherein the XRPD pattern of Form L comprises at least diffraction peaks at 2θ±0.20° of: 6.586, 8.336, 12.065, 12.568, 15.950, 17.259, 18.918, 23.483.

Preferably, the XRPD pattern of the (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate Form L comprises at least diffraction peaks at 2θ±0.20° of: 6.586, 8.336, 12.065, 12.568, 13.569, 14.503, 15.950, 17.259, 18.918, 23.483. More preferably, it further comprises diffraction peaks at 2θ±0.20° of: 5.867, 9.324, 10.609, 13.115, 17.783, 18.545, 19.713, 20.940, 21.336, 22.348, 28.165, 31.875.

Particularly preferably, the Form L has an XRPD pattern essentially as shown in FIG. 13.

In another preferred embodiment, the polymorph is Form L, and differential thermal analysis of Form L shows endothermic peaks at 192.6°C and 278.8°C (peak), and an exothermic peak at 224.2°C (peak).

In another preferred embodiment, the polymorph is Form L, and Form L has a DSC thermogram essentially as shown in FIG. 14.

In a specific embodiment of the present disclosure, a method for preparing Form L is also provided, comprising a) suspending and stirring a mixed sample of Forms A/C/K in EtOAc at room temperature with rotational mixing; b) isolating the solid by suction filtration to obtain the target Form L.

In the present disclosure, a pharmaceutical composition comprising an effective amount of the crystal form is also provided, optionally further comprising a pharmaceutically acceptable carrier.

The compositions of the present disclosure are suitable for routes of administration including oral, injectable, nasal spray, oral inhalation aerosol, and topical skin application. Preferred are nasal spray, oral inhalation aerosol, and topical skin application routes. Dosage forms include tablets, injections, parenteral preparations, sprays, aerosols, and creams, preferably sprays, aerosols, and creams.
the present disclosure also provides the use of the crystal forms and pharmaceutical compositions containing said crystal forms in the manufacture of a medicament for treating asthma, rhinitis, or neurodermatitis.

### Advantageous Effects Compared to Prior Art

The novel polymorphs of the present disclosure have characteristics such as significant efficacy, good stability, high yield, and high purity. The novel polymorphs of the present disclosure facilitate the selection and design of drug administration routes and the determination of pharmaceutical formulation process parameters, thereby improving the quality of pharmaceutical production.
the present disclosure is further described below in conjunction with specific examples. It should be understood that these examples are only for illustrating the present disclosure and are not intended to limit the scope of the present disclosure. Experimental methods without specified conditions in the following examples are generally conducted under conventional conditions or according to conditions recommended by the manufacturer. Unless otherwise indicated, percentages and parts are by weight.

Unless otherwise defined, all professional and scientific terms used herein have the same meaning as familiar to those skilled in the art. Additionally, any methods and materials similar or equivalent to those described can be applied in the methods of the present disclosure.

The experimental materials and reagents used in the following examples are commercially available unless otherwise specified.

### Example 1: Preparation of Form A

8.1 mg of starting material is weighted and put into a 1.5 mL vial, and 0.3 mL methanol is added. The sample is suspended and stirred at 5°C for 5 days. The solid is centrifuged and separated to obtain Form A.

X-ray powder diffraction testing of the obtained solid gave an XRPD pattern consistent with FIG. 1, proving the obtained product is Form A. Powder diffraction data for Form A is shown in Table 1 below. DSC testing of the obtained solid gave the result shown in FIG. 2.

**Table 1: Powder Diffraction Data for Form A**

| Diffraction Angle 2θ (°) | d-spacing (Å) | Relative Intensity |
|---|---|---|
| 5.636 | 15.68 | 10.22% |
| 8.210 | 10.77 | 12.25% |
| 8.684 | 10.18 | 4.18% |
| 11.248 | 7.87 | 100.00% |
| 13.049 | 6.78 | 7.54% |
| 14.791 | 5.99 | 2.17% |
| 15.592 | 5.68 | 3.76% |
| 16.898 | 5.25 | 10.31% |
| 17.722 | 5.00 | 11.85% |
| 17.872 | 4.96 | 9.42% |
| 18.721 | 4.74 | 6.48% |
| 19.114 | 4.64 | 3.25% |
| 21.127 | 4.21 | 4.85% |
| 22.543 | 3.94 | 0.92% |
| 24.687 | 3.61 | 3.44% |
| 25.757 | 3.46 | 0.80% |
| 28.244 | 3.16 | 0.50% |
| 29.083 | 3.07 | 0.89% |
| 30.839 | 2.90 | 1.09% |
| 31.921 | 2.80 | 0.57% |
| 36.835 | 2.44 | 0.55% |

### Example 2: Preparation of Form C

200 mg of starting material is weighted and put into a 5 mL vial, and 2 mL ethyl acetate/n-heptane (1:3, v/v) is added. The sample is suspended and stirred at room temperature for 3 days. The solid is filtered to obtain Form C.

X-ray powder diffraction testing of the obtained solid gave an XRPD pattern consistent with FIG. 3, proving the obtained product is Form C. Powder diffraction data for Form C is shown in Table 2. DSC testing of the obtained solid gave the result shown in FIG. 4.

**Table 2: Powder Diffraction Data for Form C**

| Diffraction Angle 2θ (°) | d-spacing (Å) | Relative Intensity |
|---|---|---|
| 6.463 | 13.68 | 42.14% |
| 7.402 | 11.94 | 38.11% |
| 8.988 | 9.84 | 37.65% |
| 10.812 | 8.18 | 21.46% |
| 12.000 | 7.38 | 100.00% |
| 12.898 | 6.86 | 52.25% |
| 13.088 | 6.76 | 35.37% |
| 13.611 | 6.51 | 73.08% |
| 14.328 | 6.18 | 97.94% |
| 16.233 | 5.46 | 46.85% |
| 17.205 | 5.15 | 53.97% |
| 19.794 | 4.49 | 39.95% |
| 21.032 | 4.22 | 34.72% |
| 23.376 | 3.81 | 13.60% |
| 24.173 | 3.68 | 16.71% |
| 24.815 | 3.59 | 14.69% |
| 26.038 | 3.42 | 16.45% |
| 29.921 | 2.99 | 11.58% |

### Example 3: Preparation of Form K

300 mg of starting material is weighted and put into a 5 mL vial, and 3 mL methanol is added. The sample is heated to 50°C and stirred for 3 hours, then stopped heating and allowed to cool naturally to 20°C. The solid is filtered to obtain Form K.

X-ray powder diffraction testing of the obtained solid gave an XRPD pattern consistent with FIG. 5, proving the obtained product is Form K. DSC testing of the obtained solid gave the result shown in FIG. 6.

### Example 4: Preparation of Form K

300 mg of starting material is weighted and put into a 5 mL vial, add 3 mL ethanol. The sample is heated to 50°C and stirred for 3 hours, then stopped heating and allowed to cool naturally to 20°C. The solid is filtered to obtain Form K.

X-ray powder diffraction testing of the obtained solid gave an XRPD pattern consistent with FIG. 5, proving the obtained product is Form K. Powder diffraction data for Form K is shown in Table 3.

**Table 3: Powder Diffraction Data for Form K**

| Diffraction Angle 2θ (°) | d-spacing (Å) | Relative Intensity |
|---|---|---|
| 7.148 | 12.37 | 19.58% |
| 7.750 | 11.41 | 11.26% |
| 9.336 | 9.47 | 37.23% |
| 9.955 | 8.89 | 22.15% |
| 11.239 | 7.87 | 17.80% |
| 11.639 | 7.60 | 7.34% |
| 12.769 | 6.93 | 27.25% |
| 13.826 | 6.41 | 75.14% |
| 14.315 | 6.19 | 39.39% |
| 15.581 | 5.69 | 77.59% |
| 15.960 | 5.55 | 8.61% |
| 16.748 | 5.29 | 100.00% |
| 17.864 | 4.97 | 78.36% |
| 20.050 | 4.43 | 22.30% |
| 21.198 | 4.19 | 17.02% |
| 22.587 | 3.94 | 18.18% |
| 22.909 | 3.88 | 13.75% |
| 23.725 | 3.75 | 27.66% |
| 24.383 | 3.65 | 14.04% |
| 25.100 | 3.55 | 12.56% |
| 26.611 | 3.35 | 14.10% |
| 27.920 | 3.20 | 21.50% |
| 29.369 | 3.04 | 11.35% |
| 30.269 | 2.95 | 13.13% |
| 33.349 | 2.69 | 4.09% |
| 33.909 | 2.64 | 5.18% |
| 35.012 | 2.56 | 5.79% |
| 36.306 | 2.47 | 7.22% |
| 36.978 | 2.43 | 9.31% |
| 39.174 | 2.30 | 5.20% |

### Example 5: Preparation of Form E

7.8 mg of starting material is weighted and put into a 1.5 mL vial, and 0.1 mL isopropanol is added. The sample is suspended and stirred at 5°C for 7 days. The solid is centrifuged to obtain Form E.

X-ray powder diffraction testing of the obtained solid gave an XRPD pattern consistent with FIG. 7, proving the obtained product is Form E. Powder diffraction data for Form E is shown in Table 4. DSC testing of the obtained solid gave the result shown in FIG. 8.

**Table 4: Powder Diffraction Data for Form E**

| Diffraction Angle 2θ (°) | d-spacing (Å) | Relative Intensity |
|---|---|---|
| 4.773 | 18.52 | 32.70% |
| 7.773 | 11.37 | 26.79% |
| 13.086 | 6.77 | 100.00% |
| 16.844 | 5.26 | 15.91% |
| 18.108 | 4.90 | 10.43% |
| 21.528 | 4.13 | 38.52% |

### Example 6: Preparation of Form F

10 mg of starting material is weighted into a 1.5 mL vial, and 0.05 mL N,N-dimethylformamide/water (2:1, v/v) is added. The sample is stirred at room temperature for 3 days. The solid is centrifuged to obtain Form F.

X-ray powder diffraction testing of the obtained solid gave an XRPD pattern consistent with FIG. 9, proving the obtained product is Form F. Powder diffraction data for Form F is shown in Table 5. DSC testing of the obtained solid gave the result shown in FIG. 10.

**Table 5: Powder Diffraction Data for Form F**

| Diffraction Angle 2θ (°) | d-spacing (Å) | Relative Intensity |
|---|---|---|
| 6.310 | 14.01 | 10.37% |
| 12.132 | 7.30 | 41.68% |
| 12.389 | 7.14 | 100.00% |
| 12.809 | 6.91 | 43.80% |
| 13.849 | 6.39 | 9.21% |
| 15.086 | 5.87 | 19.43% |
| 16.717 | 5.30 | 13.89% |
| 17.088 | 5.19 | 21.66% |
| 17.853 | 4.97 | 74.98% |
| 18.413 | 4.82 | 33.91% |
| 18.627 | 4.76 | 33.81% |
| 19.179 | 4.63 | 16.54% |
| 19.493 | 4.55 | 17.13% |
| 20.259 | 4.38 | 14.09% |
| 21.781 | 4.08 | 10.85% |
| 22.262 | 3.99 | 29.89% |
| 23.851 | 3.73 | 7.73% |
| 24.325 | 3.66 | 22.25% |
| 25.191 | 3.54 | 18.77% |
| 26.158 | 3.41 | 8.72% |
| 28.159 | 3.17 | 5.93% |
| 28.556 | 3.13 | 8.08% |
| 34.014 | 2.64 | 7.57% |

### Example 7: Preparation of Form I

12 mg of starting material is weighted and put into a 1.5 mL vial, and 0.2 mL N,N-dimethylformamide/n-butanol (1:1, v/v) is added. The sample is heated to 50°C and stirred until dissolved, then stopped heating and allowed to cool naturally to 20°C, then continue stirring for 12 hours. The solid is filtered to obtain Form I.

X-ray powder diffraction testing of the obtained solid gave an XRPD pattern consistent with FIG. 11, proving the obtained product is Form I. Powder diffraction data for Form I is shown in Table 6. DSC testing of the obtained solid gave the result shown in FIG. 12.

**Table 6: Powder Diffraction Data for Form I**

| Diffraction Angle 2θ (°) | d-spacing (Å) | Relative Intensity |
|---|---|---|
| 5.120 | 17.26 | 16.36% |
| 6.627 | 13.34 | 58.88% |
| 12.714 | 6.96 | 100.00% |
| 13.197 | 6.71 | 12.15% |
| 14.183 | 6.24 | 9.94% |
| 15.388 | 5.76 | 23.69% |
| 18.439 | 4.81 | 32.00% |
| 18.978 | 4.68 | 28.38% |
| 19.535 | 4.54 | 14.43% |
| 22.701 | 3.92 | 12.91% |
| 25.496 | 3.49 | 21.11% |
| 34.482 | 2.60 | 9.74% |

### Example 8: Preparation of Form L

Form C compound is added to 0.2 mL ethyl acetate until supersaturated, stirred at room temperature for 1 hour, then filtered to obtain a saturated solution. 10 mg each of Form A, C, and K compounds is added to the obtained saturated solution, and stirred at room temperature for 2 days. The solid is filtered to obtain Form L.

X-ray powder diffraction testing of the obtained solid gave an XRPD pattern consistent with FIG. 13, proving the obtained product is Form L. Powder diffraction data for Form L is shown in Table 7. DSC testing of the obtained solid gave the result shown in FIG. 14.

**Table 7: Powder Diffraction Data for Form L**

| Diffraction Angle 2θ (°) | d-spacing (Å) | Relative Intensity |
|---|---|---|
| 5.867 | 15.06 | 4.99% |
| 6.586 | 13.42 | 21.92% |
| 8.336 | 10.61 | 21.08% |
| 9.324 | 9.49 | 3.94% |
| 10.609 | 8.34 | 9.74% |
| 12.065 | 7.34 | 100.00% |
| 12.568 | 7.04 | 24.80% |
| 13.115 | 6.75 | 8.52% |
| 13.569 | 6.53 | 18.00% |
| 14.503 | 6.11 | 17.05% |
| 15.950 | 5.56 | 22.21% |
| 17.071 | 5.19 | 48.39% |
| 17.259 | 5.14 | 42.14% |
| 17.783 | 4.99 | 13.30% |
| 18.545 | 4.78 | 17.78% |
| 18.918 | 4.69 | 52.17% |
| 19.713 | 4.50 | 9.19% |
| 20.940 | 4.24 | 5.57% |
| 21.336 | 4.16 | 5.00% |
| 22.348 | 3.98 | 8.42% |
| 23.483 | 3.79 | 21.88% |
| 25.428 | 3.50 | 4.69% |
| 26.0238 | 3.42 | 3.77% |
| 26.604 | 3.35 | 5.78% |
| 27.2967 | 3.27 | 4.59% |
| 28.165 | 3.17 | 4.01% |
| 30.465 | 2.93 | 4.41% |

### Example 9: Study of Thermodynamic Stability Relationship

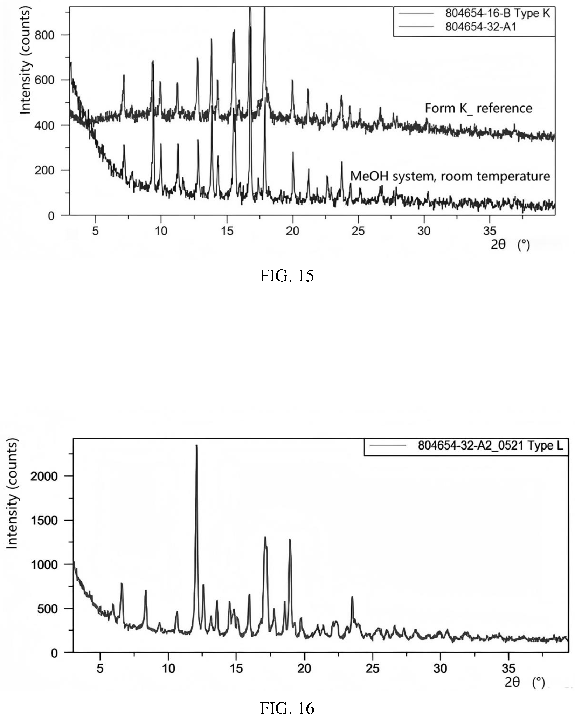

The thermodynamic stability relationship among Forms A/C/K of the present disclosure was studied at room temperature by suspension competition experiments in MeOH and EtOAc systems. First, saturated solutions of Form C in MeOH and EtOAc were prepared. Then, an equal mass ratio (approx. 10 mg each) of a mixed sample of Forms A/C/K was added to 0.2 mL of the saturated solution to form a suspension. The suspension was magnetically stirred (~600 rpm) at room temperature for 2 days, then centrifuged and tested by XRPD. The results are shown in FIGS. 15 and 16. Form K was obtained in MeOH, and Form L was obtained in the EtOAc system. Based on the above data, Form K is the thermodynamically more stable anhydrous form at room temperature.

### Example 10: DVS Testing

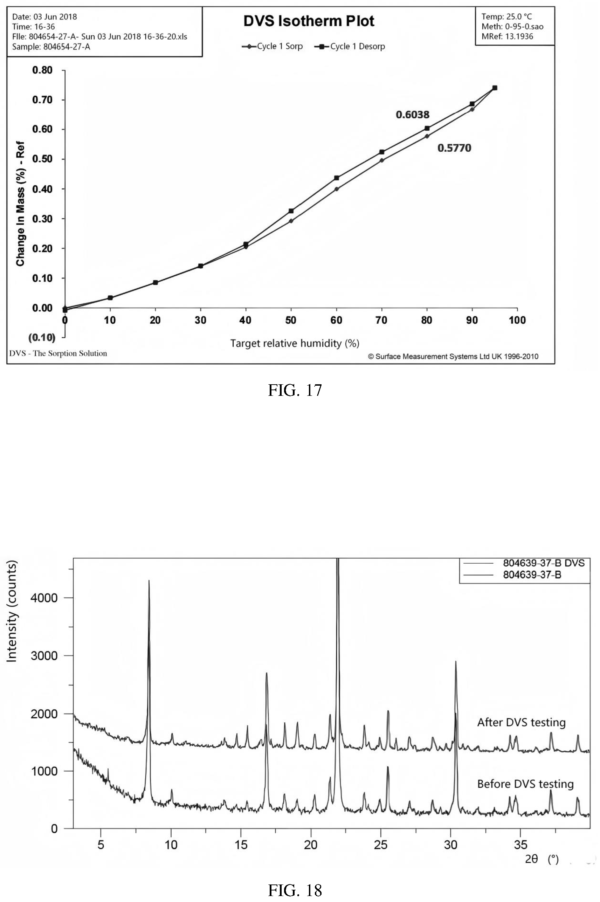

Dynamic Vapor Sorption (DVS) testing was performed on Form K of the present disclosure. The results showed that the sample absorbed 0.58% water at 25°C/80%RH, indicating that Form K has slight hygroscopicity. The crystal form did not change before and after DVS testing. The XRPD overlay of Form K before and after DVS testing is shown in FIG. 18.

### Example 11: Stability Study

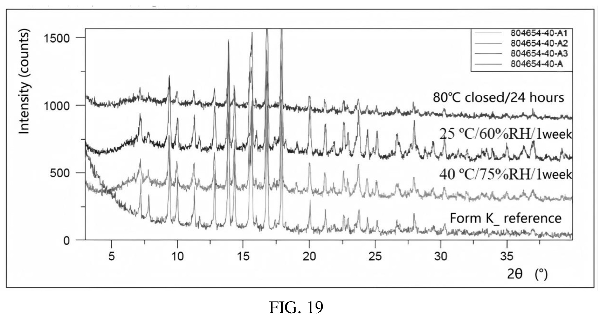

A stability study was conducted on Form K of the present disclosure. The results showed that Form K of the present disclosure did not undergo polymorphic transformation during the stability test (XRPD overlay before and after storage is shown in FIG. 19). Furthermore, no chemical degradation occurred, and it is stable at room temperature, meeting the requirements for pharmaceuticals and formulations. Details are shown in Table 8.

**Table 8: Stability Test**

| Condition | Initial Form | | Result | |
|---|---|---|---|---|
| | Form | Purity | Form | Relative Purity* |
| 80°C, closed/24 hours | | | Form K | 100.1% |
| 25°C/60%RH/1 week | Form K | 97.17% | Form K | 99.9% |
| 40°C/75%RH/1 week | | | Form K | 100.0% |

| | | | | |
|---|---|---|---|---|
| * Relative Purity = Final Purity / Initial Purity | | | | |

All documents mentioned in the present disclosure are incorporated by reference in this application as if each individual document was incorporated by reference. Furthermore, it should be understood that after reading the above teachings of the present disclosure, those skilled in the art can make various changes or modifications, and these equivalent forms also fall within the scope defined by the appended claims.

## Claims

1. A polymorph of a compound represented by formula (I), wherein the polymorph is selected from the group consisting of Form K, Form A, Form C, Form E, Form F, Form I, or Form L, 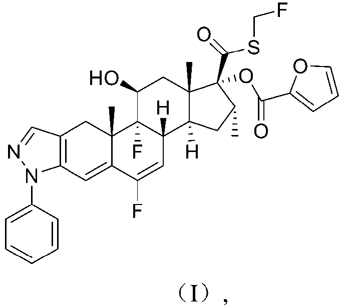 wherein the polymorph is a crystal form of (1R,2R,3aS,3bR,10aS,10bR,11S,12aS)-5,10b-difluoro-1-(((fluoromethyl)thio)carbony l)-11-hydroxy-2,10a,12a-trimethyl-7-phenyl-1,2,3,3a,3b,7,10,10a,10b,11,12,12a-dode cahydrocyclopenta[5,6]naphtho[1,2-f]indazol-1-yl furan-2-carboxylate.

2. The polymorph according to claim 1, wherein the XRPD pattern of the Form K comprises 3 or more (such as 4, 5, or 6) diffraction peaks having 2θ selected from the group consisting of: 7.148±0.20°, 9.336±0.20°, 9.955±0.20°, 11.239±0.20°, 12.769±0.20°, 13.826±0.20°, 14.315±0.20°, 15.581±0.20°, 15.960±0.20°, 16.748±0.20°, 17.864±0.20°, 20.050±0.20°, 21.198±0.20°, 22.587±0.20°, 22.909±0.20°, 23.725±0.20°, 24.383±0.20°, 26.611±0.20°, 27.920±0.20°.

3. The polymorph according to claim 1, wherein the Form K further comprises 2θ diffraction peaks selected from the group consisting of: 7.750±0.20°, 11.639±0.20°, 25.100±0.20°, 29.369±0.20°, 30.269±0.20°, 36.306±0.20°, 36.978±0.20°.

4. The polymorph according to claim 1, wherein the Form K further comprises one or more features selected from the group consisting of:
(i) the XRPD pattern of Form K is essentially as depicted in FIG. 5;
(ii) the DSC thermogram of Form K is essentially as depicted in FIG. 6;
(iii) the DSC thermogram of Form K shows an endothermic peak in the range of 286.4°C to 290.4°C;
(iv) form K is an anhydrate.

5. The polymorph according to claim 1, wherein the XRPD pattern of the Form A comprises 3 or more (such as 4, 5, or 6) diffraction peaks having 2θ selected from the group consisting of: 5.636±0.20°, 8.210±0.20°, 11.248±0.20°, 13.049±0.20°, 16.898±0.20°, 17.722±0.20°.

6. The polymorph according to claim 1, wherein the XRPD pattern of the Form A comprises 2θ diffraction peaks selected from the group consisting of: 8.684±0.20°, 14.791±0.20°, 15.592±0.20°, 17.872±0.20°, 18.721±0.20°, 21.127±0.20°, 22.543±0.20°, 24.687±0.20°, 25.757±0.20°.

7. The polymorph according to claim 1, wherein the Form A further comprises one or more features selected from the group consisting of:
(i) the XRPD pattern of Form A is essentially as depicted in FIG. 1;
(ii) the DSC thermogram of Form A is essentially as depicted in FIG. 2;
(iii) the DSC thermogram of Form A shows endothermic peaks in the ranges of 211.5°C-215.5°C and 269.6°C-273.6°C.

8. The polymorph according to claim 1, wherein the XRPD pattern of the Form C comprises 3 or more (such as 4, 5, or 6) diffraction peaks having 2θ selected from the group consisting of: 6.463±0.20°, 7.402±0.20°, 8.988±0.20°, 12.000±0.20°, 12.898±0.20°, 13.088±0.20°, 13.611±0.20°, 14.328±0.20°, 16.233±0.20°, 17.205±0.20°, 19.794±0.20°, 21.032±0.20°.

9. The polymorph according to claim 1, wherein the XRPD pattern of the Form C comprises 2θ diffraction peaks selected from the group consisting of: 10.812±0.20°, 23.376±0.20°, 24.173±0.20°, 24.815±0.20°, 26.038±0.20°, 29.921±0.20°.

10. The polymorph according to claim 1, wherein the Form C further comprises one or more features selected from the group consisting of:
(i) the XRPD pattern of Form C is essentially as depicted in FIG. 3;
(ii) the DSC thermogram of Form C is essentially as depicted in FIG. 4;
(iii) the DSC thermogram of Form C shows an endothermic peak in the range of 254.2°C-258.2°C.

11. The polymorph according to claim 1, wherein the XRPD pattern of the Form E comprises 3 or more (such as 4, 5, or 6) diffraction peaks having 2θ selected from the group consisting of: 4.773±0.20°, 7.773±0.20°, 13.086±0.20°, 21.528±0.20°.

12. The polymorph according to claim 1, wherein the Form E further comprises 2θ diffraction peaks selected from the group consisting of: 16.844±0.20°, 18.108±0.20°.

13. The polymorph according to claim 1, wherein the Form E further comprises one or more features selected from the group consisting of:
(i) the XRPD pattern of Form E is essentially as depicted in FIG. 7;
(ii) the DSC thermogram of Form E is essentially as depicted in FIG. 8;
(iii) the DSC thermogram of Form E shows endothermic peaks in the ranges of 67.2°C-71.2°C, 114.5°C-118.5°C, 156.3°C-160.3°C, and 259.5°C-263.5°C.

14. The polymorph according to claim 1, wherein the XRPD pattern of the Form F comprises 3 or more (such as 4, 5, or 6) diffraction peaks having 2θ selected from the group consisting of: 12.132±0.20°, 12.389±0.20°, 12.809±0.20°, 15.086±0.20°, 17.853±0.20°, 18.413±0.20°, 18.627±0.20°, 22.262±0.20°, 24.325±0.20°, 25.191±0.20°.

15. The polymorph according to claim 1, wherein the XRPD pattern of the Form F further comprises 2θ diffraction peaks selected from the group consisting of: 6.310±0.20°, 13.849±0.20°, 16.717±0.20°, 17.088±0.20°, 19.179±0.20°, 19.493±0.20°, 20.259±0.20°, 23.851±0.20°, 26.158±0.20°, 28.159±0.20°, 28.556±0.20°, 34.014±0.20°.

16. The polymorph according to claim 1, wherein the Form F further comprises one or more features selected from the group consisting of:
(i) the XRPD pattern of Form F is essentially as depicted in FIG. 9;
(ii) the DSC thermogram of Form F is essentially as depicted in FIG. 10;
(iii) the DSC thermogram of Form F shows endothermic peaks in the ranges of 103.3°C-107.3°C and 257.9°C-261.9°C.

17. The polymorph according to claim 1, wherein the XRPD pattern of the Form I comprises 3 or more (such as 4, 5, or 6) diffraction peaks having 2θ selected from the group consisting of: 5.120±0.20°, 6.627±0.20°, 12.714±0.20°, 15.388±0.20°, 18.439±0.20°, 18.978±0.20°, 25.496±0.20°.

18. The polymorph according to claim 1, wherein the XRPD pattern of the Form I further comprises 2θ diffraction peaks selected from the group consisting of: 13.197±0.20°, 14.183±0.20°, 19.535±0.20°, 22.701±0.20°, 34.482±0.20°.

19. The polymorph according to claim 1, wherein the Form I further comprises one or more features selected from the group consisting of:
(i) the XRPD pattern of Form I is essentially as depicted in FIG. 11;
(ii) the DSC thermogram of Form I is essentially as depicted in FIG. 12;
(iii) the DSC thermogram of Form I shows an endothermic peak in the range of 108.4°C-112.4°C.

20. The polymorph according to claim 1, wherein the XRPD pattern of the Form L comprises 3 or more (such as 4, 5, or 6) diffraction peaks having 2θ selected from the group consisting of: 6.586±0.20°, 8.336±0.20°, 12.065±0.20°, 12.568±0.20°, 13.569±0.20°, 14.503±0.20°, 15.950±0.20°, 17.259±0.20°, 18.918±0.20°, 23.483±0.20°.

21. The polymorph according to claim 1, wherein the XRPD pattern of the Form L further comprises 2θ diffraction peaks selected from the group consisting of: 5.867±0.20°, 9.324±0.20°, 10.609±0.20°, 13.115±0.20°, 17.783±0.20°, 18.545±0.20°, 19.713±0.20°, 20.940±0.20°, 21.336±0.20°, 22.348±0.20°, 28.165±0.20°, 31.875±0.20°.

22. The polymorph according to claim 1, wherein the Form L further comprises one or more features selected from the group consisting of:
(i) the XRPD pattern of Form L is essentially as depicted in FIG. 13;
(ii) the DSC thermogram of Form L is essentially as depicted in FIG. 14;
(iii) the DSC thermogram of Form L shows endothermic peaks in the ranges of 190.6°C-194.6°C and 276.8°C-280.8°C.

23. A pharmaceutical composition, wherein it comprises the polymorph of formula (I) according to any one of claims 1-22, and optionally, a pharmaceutically acceptable carrier.

24. The pharmaceutical composition according to claim 23, wherein the composition is suitable for oral, injectable, nasal spray, oral inhalation aerosol, and topical skin application routes of administration.

25. The pharmaceutical composition according to claim 23, wherein the composition is suitable for nasal spray, oral inhalation aerosol, and topical skin application routes of administration.

26. The pharmaceutical composition according to claim 23, wherein the composition can be formulated as tablets, injections, parenteral preparations, sprays, aerosols, and creams.

27. The pharmaceutical composition according to claim 23, wherein the composition can be formulated as sprays, aerosols, and creams.

28. Use of the polymorph according to any one of claims 1-22, wherein it is for the manufacture of a medicament for the treatment or prevention of rhinitis, asthma, or neurodermatitis.

29. Use of the pharmaceutical composition according to any one of claims 23-27, wherein it is for the manufacture of a medicament for the treatment or prevention of rhinitis, asthma, or neurodermatitis.
